(19) Europäisches Patentamt
European Patent Office
Office européen des brevets

(11) Publication number: **0 036 277**
**B1**

# (12) EUROPEAN PATENT SPECIFICATION

(45) Date of publication of patent specification: **28.08.85**

(51) Int. Cl.[4]: **A 61 K 9/50**

(21) Application number: **81300954.5**

(22) Date of filing: **06.03.81**

## (54) Covalently bonded liposome-protein-compositions and method of producing them.

(30) Priority: **12.03.80 US 129654**

(43) Date of publication of application:
**23.09.81 Bulletin 81/38**

(45) Publication of the grant of the patent:
**28.08.85 Bulletin 85/35**

(84) Designated Contracting States:
**AT BE CH DE FR GB IT LI NL SE**

(56) References cited:
**AU-A- 497 036**
**DE-A-2 650 502**

**CHEMICAL ABSTRACTS, vol. 91, 1979, page 486, no. 173134d Columbus, Ohio, U.S.A. V.P. TORCHLIN et al.: "Preservation of antimyosin antibody activity after covalent coupling to liposomes"**

**CHEMICAL ABSTRACTS, vol. 90, 1979, page 199, no. 99242y Columbus, Ohio, U.S.A. V.P. TORCHLIN et al.: "Comparative studies on covalent and non-covalent immobilization of protein molecules on the surface of liposomes"**

**CHEMICAL ABSTRACTS, vol. 90, 1979, page 234, no. 68851s Columbus, Ohio, U.S.A. V.P. TORCHILIN et al.: "Protein binding to liposomes"**

(73) Proprietor: **THE REGENTS OF THE UNIVERSITY OF CALIFORNIA**
**2200 University Avenue**
**Berkeley, California 94720 (US)**

(72) Inventor: **Papahadjopoulos, Demetrios P.**
**3170 Condit Road**
**Lafayette California 94549 (US)**
Inventor: **Heath, Timothy D.**
**478 Warren Drive, No. 307**
**San Francisco California 94131 (US)**

(74) Representative: **Harrison, David Christopher et al**
**MEWBURN ELLIS & CO 2/3 Cursitor Street**
**London EC4A 1BQ (GB)**

(56) References cited:
**CHEMICAL ABSTRACTS, vol. 81, 1974, page 370, no. 150043c Columbus, Ohio, U.S.A. C.R. ALVING et al.: "Comparative properties of four galactosyl lipids as antigens in liposomes"**
**"ULTROGEL AND MAGNOGEL", Practical guide for use in affinity chromatography, 1979, Pharmindustrie Clichy, FR.**

**CHEMICAL ABSTRACTS, vol. 75, 1971, page 206, 86669h Columbus, Ohio, U.S.A. C.J. SANDERSON et al.: "Simple method for coupling proteins to insoluble polysaccharides"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 036 277 B1

(56) References cited:

**THE JOURNAL OF BIOLIGICAL CHEMISTRY, vol. 255, no. 21, 10th November 1980, pages 10509-10516 U.S.A. D.L. URDAL et al.: "Tumor-associated ganglio-N-triosylceramide"**

**The file contains technical information submitted after the application was filed and not included in this specification**

**Description**

The present invention relates generally to liposomes, and more particularly to liposomes which encapsulate materials, such as drugs, nucleic acids, proteins and the like, and which are efficiently covalently bound to a variety of biologically active proteins without rupture of the liposomes.

Liposomes are now well recognized as useful for delivery of therapeutic agents, such as cytotoxic drugs or other macromolecules capable of modifying cell behaviour, to *in vivo* sites. For example, U.S. Patent 3,993,754, inventors Rahman, et al, issued November 23, 1976, discloses an improved method for chemotherapy of malignant tumors in which an antitumor drug is encapsulated within liposomes and the liposomes containing the encapsulated drug are injected into an animal or man.

It has been suggested that target, or *in vivo* site, specificity might be conferred on liposomes by their association with specific antibodies or lectins. Methods of associating antibodies with liposomes have been described and may be generally divided into two groups — nonspecific association and covalent attachment.

Nonspecific association appears to rely upon the affinity of the Fc portion of the antibody for the hydrophobic region of the lipid bilayer. This has little practical value because the liposomes are rendered more permeable to their encapsulated contents and may themselves be aggregated. Further, it is not known how stable this complex would be in plasma.

Previous attempts to covalently attach protein to liposomes have been unsatisfactory. For example, some of these prior attempts to covalently attach protein to liposomes have involved modifications of the proteins. This is disadvantageous because such protein modifications have tended to denature the protein, and thus a substantial loss of biological activity has ensued. DE—A—2650502, *Chemical abstracts* 90 (1979), 99242y and *Chemical abstracts* 91 (1979) 173134d, all disclose the use of an amino-reactive bifunctional agent in reaction between a protein and a liposome. This technique is standard in protein-protein coupling, but does not give any significant covalent coupling between a liposome and a protein — any covalent coupling will be at a level of less than 20 mg/micromole.

It is a further problem that any coupling technique used must be such that the physical integrity of the liposomes is not harmed.

The present invention provides a method for activating liposomes by the oxidation of oxidisable polar heads of lipids of the liposomes to an aldehyde moiety. This production of aldehyde moieties, to allow covalent coupling onto them of proteins which do not need prior modification, has been disclosed per se in *Chemical Abstracts* 75 (1971), 86669h, but in the context of coupling of proteins onto insoluble polysaccharides.

The present invention also provides covalently bonded liposome-protein compositions in which the amount of protein so bound is at least 40 mg/micromole of the lipids of the liposome, and a method of forming such compositions by the coupling of the protein to the activated liposome in a polar solvent in the presence of sodium cyanoborohydride. Materials encapsulated within the liposome, remain inside them; the selected proteins are coupled to the outer surface.

Although it is applicable to any protein having primary or secondary amino groups, the invention is of particular value in that it allows the binding to liposomes of proteins such as IgG and $F[ab']_2$. The biological activity of the proteins remains significant.

The liposome-protein compositions may be used for selective targetting to specific cell types and tissues or in cell agglutination.

Precursor, that is non-activated, liposomes useful as the starting liposomes in the present invention may be prepared by any of various conventional methods known to the art. These various known methods may be generally characterized as yielding either unilamellar vesicles or multilamellar vesicles. Either liposomal structure is suitable for the present invention. However, due to the generally larger internal space available in unilamellar liposomes, the precursor liposomes of the present invention are preferably prepared by the reverse-phase evaporation vesicle (REV) method as is described in *Proc. Natl. Acad. Sci. U.S.A.*, Volume 75, No. 9, pp. 4194—4198 (1978), entitled "Procedure For Preparation of Liposomes With Large Internal Aqueous Space And High Capture By Reverse-Phase Evaporation", Szoka, Jr. and Papahadjopoulos, which disclosure is incorporated by herein reference.

As is known to the art, a wide variety of materials may be encapsulated by the precursor liposomes. For example, the precursor liposomes can encapsulate cytotoxic drugs, can encapsulate nucleic acids, and can encapsulate various proteins.

In any event, the precursor liposomes suitable for the present invention may be formed from either phosphatidylglycerol (hereinafter also referred to as "PG"), which has an oxidisable group at the polar head region, as the sole lipid, or may be formed from a mixture of two or more different lipids.

When formed from two or more different lipids, at least one of the lipids must contain oxidisable groups, such as vicinal amino or vicinal hydroxyl groups, along the polar head region of the lipid molecule. For example, in the instance of vicinal amino groups, a glycolipid having galactosamine or glucosamine residue is a suitable oxidisable lipid in accordance with the present invention. More usually, at least one of the lipids will have vicinal hydroxyl groups at the polar head region. Particularly preferred as one of the lipids (that is, the oxidisable lipid) in a lipid mixture are the glycolipids such as lactosyl ceraminde, galactocerebroside, gangliosides, and trihexosyl ceramide, and the phospholipids, such as phosphatidylglycerol and phosphatidylinositol.

The amount of such lipids having oxidisable groups (generally herein referred to as "oxidisable lipids") may vary with respect to the total lipids forming the precursor liposomes; however, it is preferred that the mole percent of oxidisable lipids be in an amount of at least about 10 mole percent with respect to a total of the mixture of lipids.

Particularly preferred amounts of oxidisable lipids with respect to the total lipids are illustrated by Table I, below.

TABLE I

| Oxidisable Lipid | Mole of Oxidisable Lipid To Total Lipid Mixture |
|---|---|
| Lactosylceramide | About 10 |
| Trihexosylceramide | About 10 |
| Galactocerebroside | About 20 |
| Phosphatidylglycerol | About 33—40 |
| Phosphatidylinositol | About 20 |
| Gangliosides | About 10 |

The structures of the preferred oxidisable lipids are well known; however, for clarity Figure 1, below, illustrates PG as representative of the general structures of the oxidisable lipids having the polar head regions and the region of non-polar tails.

FIGURE I: PG

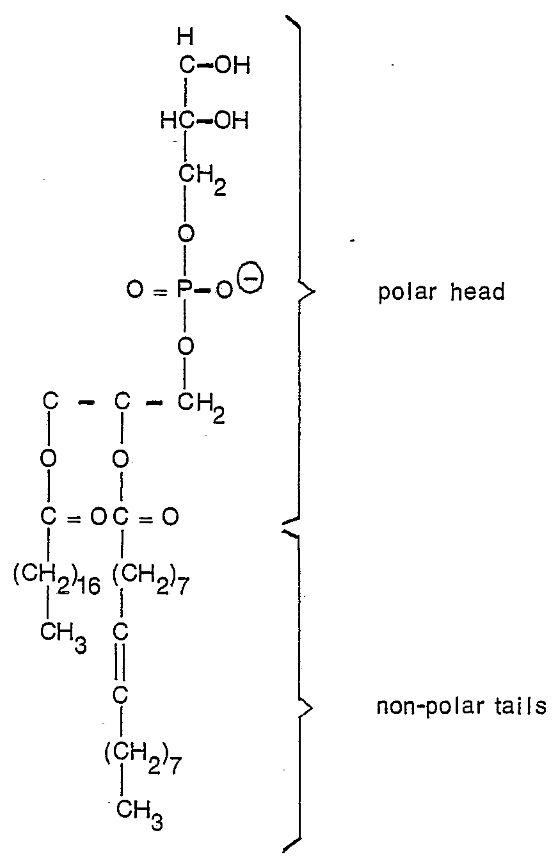

Figure 1 is generally representative of all of the lipids which may be mixed to form the precursor liposomes in defining the polar head region and the non-polar tails. The Figure 1 structure is more particularly generally representative of the oxidisable lipids which have vicinal hydroxyl groups at the polar head region thereof.

When a mixture of lipids, including the oxidisable lipid, is utilized to form the precursor liposomes, then the remaining lipid or lipids may include any of the amphiphilic substances known to produce liposomes. A particularly preferred lipid for combination with the oxidisable lipids is phosphatidylcholine (hereinafter also referred to as "PC"), sphingomyelin or mixtures thereof.

As is known, the above discussed mixtures of lipid molecules form precursor liposomes with the lipid molecules being arranged in either one bimolecular layer (unilamellar) or a plurality of bimolecular layers (multilamellar). In any event, the most outward bimolecular layer forms an outer surface for the liposome. In an aqueous solution, the polar head regions of the lipid molecules are exposed, or extend into, the aqueous system in a generally radially outward orientation with respect to the outer surface. The non-polar tails extend radially inwardly with respect to the outer surface and form a substantially continuous hydrocarbon phase of the bimolecular layer. This substantially continuous hydrocarbon phase is relatively impermeable, and acts to encapsulate the materials inside the precursor liposomes.

Nevertheless, some mixtures of lipids forming the precursor liposomes may tend to be permeable to small molecules, and cholesterol is a desirable addition to some of these lipid mixtures for reducing the permeability of the precursor liposomes. The cholesterol tends to orientate within the bi-molecular layer. Other components may be utilized in place of cholesterol to reduce the liposome permeability. For example, a phosphatidyl choline having the fatty acid saturated aliphatic chain, or non-polar tails, of a length of 18 (rather than the usual unsaturated 16 to 18 carbon chain obtainable from egg yolks) may be utilized. However, when sphingomyelin is mixed with the oxidisable lipid, the precursor liposomes thereof are inherently quite impermeable to small molecules.

Turning to the invention, a solution of precursor liposomes is provided as has been described above. This solution is preferably a polar solution, such as an aqueous solution, but may also be a non-polar solution. The precursor liposomes are contacted with a sufficient amount of a relatively mild oxidizing reagent to produce activated liposomes. Where the lipids to be used for liposomes are in a non-polar solution, the oxidizing reagent may be lead tetraacetate. In the preferred polar solution, the oxidizing reagent of the contacting step is a periodate reagent, usually sodium periodate, which cleaves the vicinal amino or hydroxyl groups at the polar head regions of the oxidisable lipids.

Where the solution is polar and the oxidizing agent is a periodate reagent, the pH and osmolarity of the liposome solution and an added amount of periodate reagent should be substantially the same. The pH is typically about 6.0 to about 8.5. The oxidizing reagent produces activated liposomes by oxidizing the oxidisable groups, such as the vicinal hydroxyl or amino groups of the oxidisable lipid, to yield aldehyde moieties at the polar head regions of the oxidisable lipids. A sufficient quantity of periodate reagent will usually be a molar ratio with respect to the total of lipid molecules of from about 1.5:1 to about 6:1. The oxidation reaction of the contacting step is typically left to proceed for about one-half hour at room temperature, although the reaction may be permitted to proceed for up to about one hour on ice. The periodate reagent is then preferably removed by gel filtration through a column of dextran polymeric beads having an exclusion limit of about 75,000 daltons.

Reaction schemes I, II and III diagrammatically illustrate the activation of precursor liposomes, with the oxidisable lipids being phosphatidylglycerol, phosphatidylinositol and lactosylceramide respectively.

REACTION SCHEME I

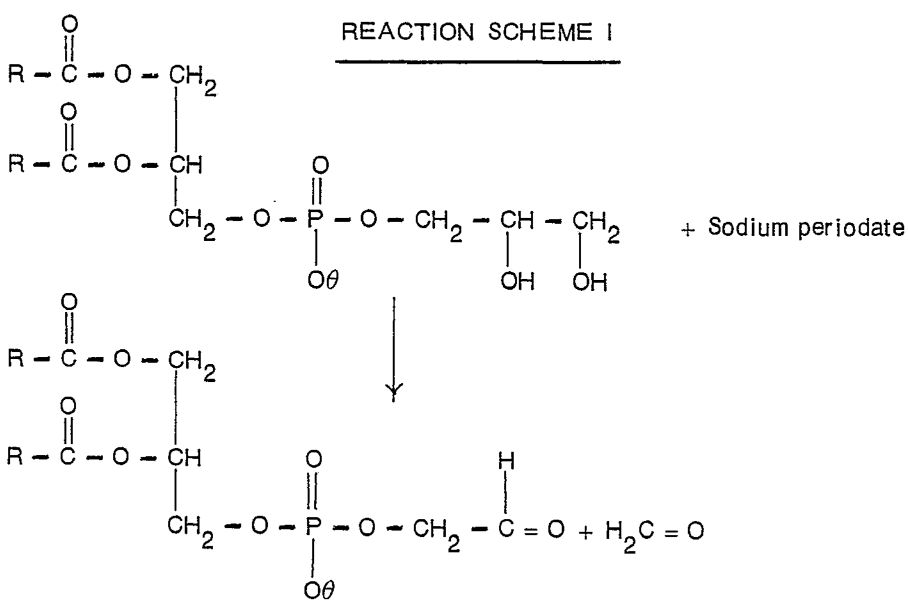

REACTION SCHEME II

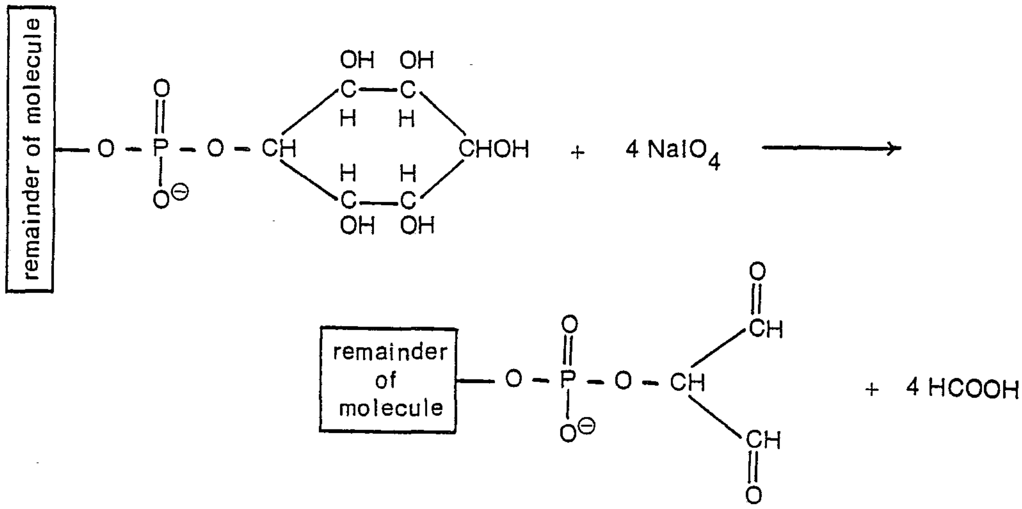

REACTION SCHEME III

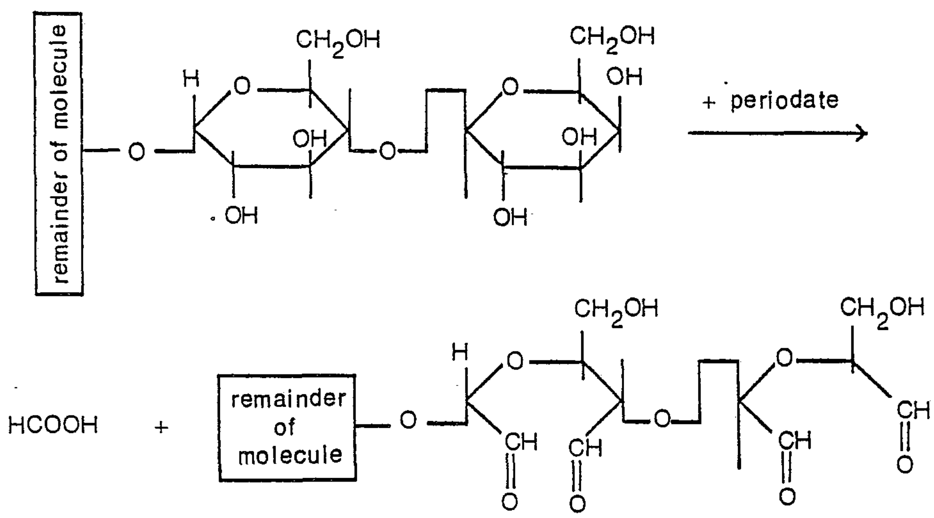


Substantially all of the material which is interior the outer surfaces of the activated liposomes remains encapsulated during the above-described oxidation with periodate reagent. As illustrated by Reaction Schemes I—III, the aldehyde moieties which are formed by the oxidation, or modification, of the oxidisable lipids at the polar head regions thereof define covalent binding sites for the protein to be bound, or coupled.

A wide variety of proteins may be attached, or coupled, to the activated liposomes. The mechanism of coupling is believed to occur between the primary or secondary amino group along the protein and the aldehyde moiety of the activated liposomes so as to form a Schiff-base, for example, with the primary amino group of a lysyl moiety. Such a mechanism is diagrammatically represented by Reaction Scheme IV, which for simplicity illustrates only the terminal galactose (after modification) of lactosylceramide.

REACTION SCHEME IV

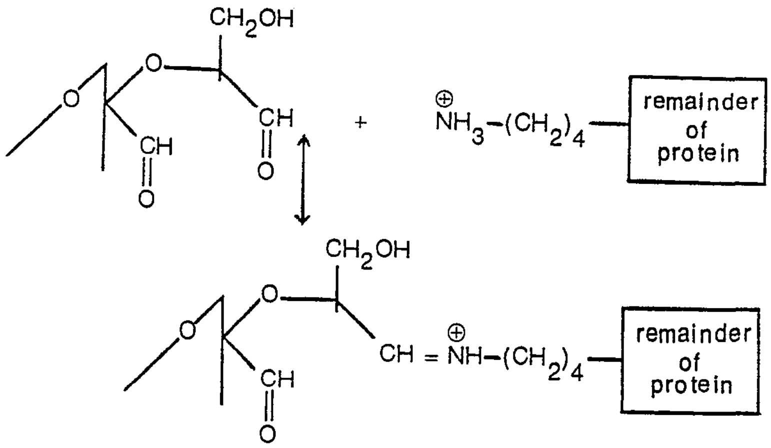

The coupling is driven to completion by a mild reducing agent, preferably sodium cyanoborohydride, so that a stable, covalent bond is formed between the protein and the activated liposome. For example, addition of a sufficient amount of sodium cyanoborohydride drives the Schiff-base of reaction scheme IV above, to completion, as is generally illustrated by Reaction Scheme V, below.

REACTION SCHEME V

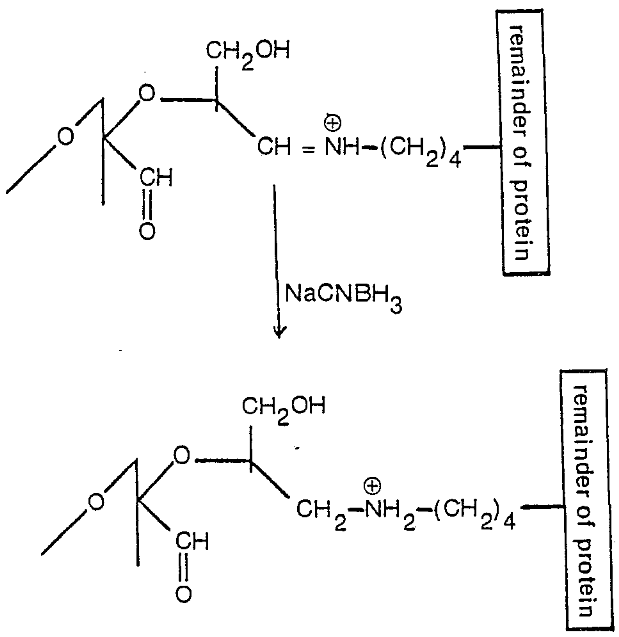


Reaction Schemes IV and V, above, diagrammatically illustrate coupling of a protein with lactosylceramide, where the lactosylceramide has been modified by oxidation to include aldehyde moieties. Use of the other oxidisable lipids proceeds by an analogous manner. In the instance of modified lactosylceramide, the secondary amine moiety which is covalently binding the protein to the activated liposome may further proceed, in the presence of sodium cyanoborohydride, into an even more stable, tertiary amine form.

Although sodium cyanoborohydride is the preferred reducing agent for coupling proteins to the activated liposomes, other reducing agent may be utilized, depending upon the particular circumstances. For example, borohydride may be utilized; however, the coupling reaction would usually then be conducted at a relatively alkaline pH, which may tend to denature the protein being coupled.

Suitable proteins for adequate coupling will have at least one primary or secondary amino group, and preferably a plurality of primary or secondary groups. Proteins having at least one and preferably at least about 20 lysyl moieties per molecule are more preferred. IgG, with about 60 lysyl moieties, has been found to be particularly well coupled; another preferred antibody for coupling with the activated liposome is $F[ab']_2$.

Four aqueous solutions of liposome, each containing from about 10 to about 40 micromoles of lipid per milliliter, were activated as previously described. The precursor liposomes had been prepared by the REV procedure and had been extruded through a polycarbonate filter to yield liposomes having a diameter of about 0.2 micron. The solutions were buffered at a pH of from about 6.0 to about 8.5. A fifth liposome solution, wherein the oxidisable lipid was not oxidized, was prepared as a control. These four activated liposome solutions in accordance with the present invention and the fifth control solution were as illustrated by Table II.

TABLE II

| Solution # | Lipid Composition/ Molar Ratio | Total Lipid (micromole) | Vol. (ml) |
|---|---|---|---|
| 1 | PC/Lactosylceramide, 10:1 | 9.21 | 2.8 |
| 2 | PC/Trihexosylceramide, 10:1 | 16.44 | 4.5 |
| 3 | PC/PG, 1:1 | 9.5 | 3.1 |
| 4 | Galactocerebroside/PC/ Cholesterol, 2:4:5 | 15 | 0.6 |
| 5* | PC/Lactosylceramide, 10:1 | 7.11 | 2.8 |

*Control solution, liposomes not activated

The five solutions as in Table II were treated as follows. 5 to 10 milligrams of IgG in the same buffer as the liposome solutions was added to the respective liposome solutions (the activated liposomes were suspended in the solutions with substantially no clumping). Sufficient sodium cyanoborohydride was added to give a concentration of about 20 millimolar, and the solutions were left for about 2 to about 3 hours at room temperature. The liposomes having covalently bound IgG thereon were then purified by conventional methods, such as column gel filtration or centrifugation. The amount of coupling is illustrated by Table III, below (the number of molecules per vesicle was estimated on the assumption that the vesicles were 0.2 micron in diameter, with about $1.8 \times 10^{12}$ vesicles/micromole lipid).

TABLE III

| Liposome Solution | IgG (mg) added | Protein: Lipid Ratio ($\mu g / \mu$ mole) | ($\mu g$ / mg lipid) | Molecules IgG/vesicle |
|---|---|---|---|---|
| 1 | 20 | 112 | 147 | 251 |
| 2 | 14 | 57 | 75 | 128 |
| 3 | 14 | 47 | 62 | 106 |
| 4 | 10 | 96 | 126 | 216 |
| 5* | 20 | 11 | 14 | 25 |

* Control solution, liposomes not activated.

IgG coupling under the above described conditions has typically resulted in the binding of from about 50 to about 200 micrograms of IgG per micromole of lipid. Substantially no coupling is observed if the liposomes have not been activated. Nonspecific binding of proteins to activated liposomes was below the limits of the protein assay utilized in determining coupling.

The proteins which may be covalently bound, or coupled, to the activated liposomes preferably exhibit biological activity. The coupling process in accordance with the present invention preserves a significant

amount of the biological activity of the coupled protein. This is illustrated in two separate ways by use of immunopurified rabbit antifluorescein antibody, as follows.

Antifluorescein IgG binds specifically to fluorescein isothiocyanate and carboxyfluorescein. Upon binding to the antibody, the fluorescence of the fluorescein is abolished, and this was used to measure the binding activity of the antibody. Successive additions of antibody to a solution of carboxyfluorescein reduced the fluorescence due to quenching of the fluorophore upon binding to the antibody. The antigen binding capacity of liposome-bound antibody was compared by correlating the percentage reduction in fluorescence for a variety of specified protein concentrations in linear ranges where quenching was proportional to the protein concentration as illustrated by Table IV, below (wherein the original antibody, or control, linear range was from about 78/1 to about 30/4; unbound antibody was from about 85/1 to about 40/5; and coupled antibody-activated liposomes was linear over the entire range illustrated).

TABLE IV

Fluorescent Intensity/Antibody conc. × $10^8$ (μmole/ml)

| Original Antibody (Control) | Unbound Antibody | Coupled Antibody-Activated Liposome |
|---|---|---|
| 78/1 | 85/1 | 95/1 |
| 60/2 | 75/2 | 90/2 |
| 40/3 | 62/3 | 85/3 |
| 30/4 | 50/4 | 78/4 |
| 18/5 | 40/5 | 70/5 |
| 12/6 | 35/6 | 65/6 |
| 8/7 | 30/7 | 60/7 |
| 8/8 | 20/8 | 58/8 |
| | 15/9 | 50/9 |

As illustrated by Table IV, above, the fluorescent quenching of carboxyfluorescein by the original antibody preparation (control) and the antibody that was recovered from the coupling process may be compared to antibody bound to the activated liposomes. If the activity of the original, control preparation is set at 100%, then the activity of the activated liposome bound antibody is about 32%, and of the recovered antibody is about 70%. Antigen binding capacity is, therefore, only partially inhibited by the inventive coupling process, and the coupled protein displays, or retains, a significant amount of antigen binding capacity.

Antibody activity of the coupled antibody was also assessed by the ability of the coupled antibody to agglutinate erythrocytes. The activated liposomes conjugated with antigen-antibody were incubated with erythrocytes conjugated with fluorescein-isothiocyanate. This resulted in the agglutination of the erythrocytes and the haemagglutinating titre was marginally increased by the antibodies which were coupled to activated liposomes. This is illustrated by Table V, below.

TABLE V

Haemagglutination of FITC-Human Erythrocytes by Rabbit Antifluorescein IgG

| Preparation | Titre (microgram/ml) |
|---|---|
| Liposomes Bound Antibody (500 molecules/vesicle) | 1.22 |
| Untreated Antibody | 1.92 |

The titre is expressed as the minimum concentration observed to cause agglutination. Lower values indicated more effective agglutinating capacity.

The data of Table VI, below, demonstrates that the encapsulated carboxyfluorescein did not leak from

PG:PC:cholesterol (1 to 1 to 1 molar ratios) REV liposomes upon activation thereof with periodate and during subsequent protein attachment procedures.

TABLE VI

| Sample | Carboxyfluorescein/ Lipid Ratio (mole/mole) | % or original |
|---|---|---|
| Untreated Liposomes | 0.260 | 100 |
| Periodate Oxidized/ Desalted | 0.260 | 100 |
| Protein Coupled and Separated | 0.286 | 104 |

Thus Table VI illustrates that the coupling does not cause the release of the encapsulated material of the activated liposomes.

In summary, the new method provides the opportunity of increasing relative uptake of material encapsulated by the liposomes coupled to protein by specific cells. Since the liposomes can be loaded with cytotoxic drugs or other macromolecules capable of modifying cell behaviour, such an increased specificity could be an important advantage for the selective targeting of various molecules to specific cell types and tissues. Such targeting could result in a substantial increase of the therapeutic index of a variety of cytotoxic drugs. In addition, such increased specificity could be used for cellular delivery of nucleic acids in gene therapy, and for delivery of proteins in enzyme replacement therapy in cases of congenital enzyme deficiencies and metabolic disorders. Possible *in vivo* targets for the lipsomes covalently bound to protein may include solid tumors or circulating cells such as lymphocytes. The delivery of cytotoxic agents by the liposomes covalently bound to protein to lymphocytes could be of great value as a specific immunosuppressant. These and other medical applications are now within the realm of feasibility because of the existence of purified monoclonal antibodies directed against specific cells, which antibodies can be readily coupled to the activated liposomes in accordance with the present invention.

**Claims**

1. A composition comprising:
a plurality of liposomes, the liposomes including lipid molecules;
protein being covalently bound to the liposomes to form coupled liposome-protein species, the bound protein of the coupled liposome-protein species being biologically active and in an amount of at least 40 micrograms per micromole of the lipid molecules.

2. A composition according to Claim 1, wherein the protein is an antibody and the biological activity thereof includes an antigen binding capacity.

3. A composition according to Claim 2, wherein the protein has an original, determinable antigen binding capacity prior to having been bound to the liposomes, and the protein of the liposome-protein species has an antigen-binding capacity of greater than 10% with respect to the original, determinable antigen binding capacity.

4. A composition according to any one of Claims 1 to 3, wherein the protein is IgG of $F[ab']_2$.

5. A composition according to Claim 4, wherein the IgG of $F[ab']_2$ has an original, determinable antigen binding capacity prior to having been bound to the liposomes, and the IgG or $F[ab']_2$ of the liposome-protein species has an antigen binding capacity of at least 32% with respect to the original, determinable antigen binding capacity.

6. A composition according to any one of Claims 1 to 4 wherein the plurality of liposomes have at least about 106 molecules of protein bound per liposome.

7. A composition according to any one of Claims 1 to 6 for use in cell agglutination.

8. A composition according to any one of the preceding Claims wherein the liposomes are unilamellar and of substantially predetermined diameter.

9. A method for producing activated liposomes characterised in that liposomes with outer surfaces including a plurality of lipid molecules, said lipid molecules having polar heads and non-polar tails and at least some of said polar heads with oxidisable groups thereat, are contacted with an oxidizing reagent to produce activated liposomes by oxidation of the oxidisable groups to aldehyde moieties.

10. A method according to Claim 9 whererin at least some of said polar heads with oxidisable groups thereat have vicinal hydroxyl groups and are selected from lactosylceramide, galactocerebroside, trihexosylceramide, phosphatidylglycerol, phosphatidylinositol, gangliosides and mixtures thereof.

11. A method according to Claim 10 wherein said lipid molecules are a mixture of lipids, the mixture

including phosphatidylcholine or sphingomyelin.

12. A method accoridng to Claim 10 or Claim 11 wherein said lipid molecules having said polar heads with vicinal hydroxyl groups are in an amount of at least about 10 molar percent with respect to a total of said mixture of lipids.

13. A method according to any one of Claims 9 to 11 wherein said lipid molecules define a bimolecular layer and said liposomes include cholesterol disposed within said bi-molecular layer.

14. A method according to any one of Claims 9 to 12 wherein said liposomes are unilamellar vesicles having substantially equivalent diameters.

15. A method according to any one of Claims 9 to 14 wherein said oxidizing reagent is a periodate reagent or is lead tetraacetate.

16. A method according to Claim 15 wherein the contacting step is conducted in a polar solvent and said oxidizing reagent is sodium periodate in a ratio with respect to the lipid molecules of from 1.5:1 to 6:1.

17. A method for coupling proteins to liposomes by:

activating liposomes, by a method according to any one of Claims 9 to 16 and

admixing a protein having at least one primary or secondary amino group with said activated liposomes, the admixing being conducted in a polar solvent in the presence of a sufficient quantity of sodium cyanoborohydride to bind covalently said protein to said activated liposomes.

18. A method according to Claim 17 wherein said protein has at least about 20 lysyl moieties therealong.

19. A method according to Claim 17 wherein said protein is IgG or $F(ab')_2$.

20. A method according to Claim 17, Claim 18, or Claim 19, wherein the protein is an antibody having an original, determinable antigen binding capacity prior to the admixing step, and said protein bound to said liposomes from the admixing step has an antigen binding capacity of not less than 10% with respect to said original, determinable antigen binding capacity.

21. A liposome covalently bound to at least 40 µg per micromole of a biologically active protein obtainable by a method as claimed in any one of Claims 17 to 20.

**Patentansprüche**

1. Zusammensetzung umfassend:

eine Vielzahl von Liposomen, welche Liposomen Lipidmoleküle einschließen;

Protein, das kovalent an die Liposome gebunden ist, um gekoppelte Liposom-Protein-Gattungen zu bilden, wobei das gebundene Protein der gekoppelten Liposom-Protein-Gattung biologisch aktiv ist und in einer Menge von wenigstens 40 µg/µMol der Lipidmoleküle vorliegt.

2. Zusammensetzung nach Anspruch 1, worin das Protein ein Antikörper ist und seine biologische Aktivität eine Antigenbindungsfähigkeit einschließt.

3. Zusammensetzung nach Anspruch 2, worin das Protein eine ursprüngliche, feststellbare Antigenbindungsfähigkeit vor der Bindung an die Liposomen besitzt und das Protein der Liposom-Protein-Gattung eine Antigenbindungsfähigkeit aufweist, die höher als 10% der ursprünglichen, feststellbaren Antigenbindungsfähigkeit ist.

4. Zusammensetzung nach einem der Ansprüche 1 bis 3, worin das Protein IgG oder $F[ab']_2$ ist.

5. Zusammensetzung nach Anspruch 4, worin das IgG oder $F[ab']_2$ eine ursprüngliche, feststellbare Antigenbindungsfähigkeit vor der Bindung an die Liposomen besitzt und das IgG oder $F[ab']_2$ der Liposom-Protein-Gattung eine Antigenbindungsfähigkeit aufweist, die wenigstens 32% der ursprünglichen, feststellbaren Antigenbindungsfähigkeit beträgt.

6. Zusammensetzung nach einem der Ansprüche 1 bis 4, worin die Vielzahl der Liposomen wenigstens etwa 106 Moleküle von gebundenem Protein pro Liposom aufweist.

7. Zusammensetzung nach einem der Ansprüche 1 bis 6 für die Verwendung bei der Zellenagglutinierung.

8. Zusammensetzung nach einem der vorhergehenden Ansprüche, worin die Liposomen unilamellar und von im wesentlichen vorgegebenem Durchmesser sind.

9. Verfahren zur Herstellung von aktivierten Liposomen, dadurch gekennzeichnet, daß Liposomen mit Außenflächen, die eine Vielzahl von Lipidmolekülen einschließen, welche Lipidmoleküle polare Kopfbereiche und nichtpolare Schwanzbereiche aufweisen und worin wenigstens an einigen der polaren Kopfbereiche oxydierbare Gruppen vorhanden sind, mit einem Oxydationsmittel in Berührung gebracht werden, um aktivierte Liposomen durch Oxydation der oxydierbaren Gruppen zu Aldehydeinheiten herzustellen.

10. Verfahren nach Anspruch 9, worin wenigstens einige der genannten polaren Kopfbereiche, an denen oxydierbare Gruppen vorhanden sind, vicinale Hydroxylgruppen aufweisen und aus Lactosylceramid, Galactocerebrosid, Trihexosylceramid, Phosphatidylglyzerin, Phosphatidylinositol, Gangliosiden und Mischungen derselben ausgewählt sind.

11. Verfahren nach Anspruch 10, worin die genannten Lipidmoleküle eine Mischung von Lipiden sind, welche Mischung Phosphatidylcholin oder Sphingomyelin einschließt.

12. Verfahren nach Anspruch 10 oder 11, worin die genannten Lipidmoleküle, die die genannten

polaren Kopfbereiche mit vicinalen Hydroxylgruppen aufweisen, in einer Menge von wenigstens etwa 10 Mol-% in bezug auf eine Gesamtmenge der genannten Mischung von Lipiden vorhanden sind.

13. Verfahren nach einem der Ansprüche 9—11, worin die genannten Lipidmoleküle eine bimolekulare Schicht bilden und die genannten Liposomen Cholesterin einschließen, das innerhalb der genannten bimolekularen Schicht eingelagert ist.

14. Verfahren nach einem der Ansprüche 9—12, worin die genannten Liposomen unilamellare Vesikel mit im wesentlichen äquivalenten Durchmessern sind.

15. Verfahren nach einem der Ansprüche 9—14, worin das genannte Oxydationsmittel ein Perjodat-Reaktionsmittel oder Bleitetraacetat ist.

16. Verfahren nach Anspruch 15, worin der Berührungsschritt in einem polaren Lösungsmittel durchgeführt wird und das genannte Oxydationsmittel Natriumperjodat in einem Verhältnis in bezug auf die Lipidmoleküle von 1,5:1 bis 6:1 ist.

17. Verfahren zum Koppeln von Proteinen an Liposomen durch

Aktivieren der Liposomen nach einem Verfahren nach einem der Ansprüche 9—16, und

Mischen eines Proteins, das wenigstens eine primäre oder sekundäre Aminogruppe aufweist, mit den genannten aktivierten Liposomen, wobei das Mischen in einem polaren Lösungsmittel in Gegenwart einer ausreichenden Menge Natriumcyanoborhydrid durchgeführt wird, um das genannte Protein kovalent an die genannten aktivierten Liposomen zu binden.

18. Verfahren nach Anspruch 17, worin das genannte Protein wenigstens etwa 20 Lysylanteile entlang seiner Länge aufweist.

19. Verfahren nach Anspruch 17, worin das genannte Protein IgG oder $F[ab']_2$ ist.

20. Verfahren nach Anspruch 17, Anspruch 18 oder Anspruch 19, worin das Protein ein Antikörper mit einer ursprünglichen, feststellbaren Antigenbindungsfähigkeit vor dem Schritt des Mischens ist, und das genannte, an die genannten Liposomen gebundene Protein vom Schritt des Mischens an eine Antigenbindungsfähigkeit von nicht weniger als 10% in bezug auf die genannte ursprüngliche, feststellbare Antigenbindungsfähigkeit besitzt.

21. Ein Liposom, das kovalent an wenigstens 40 µg pro µMol eines biologisch aktiven Proteins gebunden ist, das nach einem Verfahren nach einem der Ansprüche 17—20 erhältlich ist.

**Revendications**

1. Composition comprenant:

un certain nombre de liposomes, les liposomes comprenant des molécules de lipide;

une protéine étant liée par liaison covalente aux liposomes pour former des espèces couplées liposomes-protéine, la protéine liée de l'espèce couplée liposomes-protéine étant biologiquement active et en une quantité d'au moins 40 microgrammes par micromole des molécules de lipide.

2. Composition selon la revendication 1, où la protéine est une anticorps et son activité biologique comprend une capacité liant l'antigène.

3. Composition selon la revendication 2, où la protéine a une capacité liant l'antigène déterminable d'origine avant d'avoir été liée aux liposomes, et la protéine de l'espèce liposomes-protéine a une capacité de liaison de l'antigène de plus d'environ 10% par rapport à la capacité de liaison de l'antigène d'origine, déterminable.

4. Composition selon l'une quelconque des revendications 1 à 3, où la protéine est IgG ou $F[ab']_2$.

5. Composition selon le revendication 4, où IgG ou $F[ab']_2$ a une capacité de liaison de l'antigène d'origine déterminable avant d'avoir été liée aux liposomes et le IgG ou $F[ab']_2$ de l'espèce liposomes-protéine a une capacité de liaison de l'antigène d'au moins 32% par rapport à la capacité de liaison de l'antigène déterminable d'origine.

6. Composition selon l'une quelconque des revendications 1 à 4, où la quantité de liposomes a au moins environ 106 molécules de protéine liée par liposome.

7. Composition selon l'une quelconque des revendications 1 à 6, pour une utilisation dans l'agglutination des cellules.

8. Composition selon l'une quelconque des revendications précédentes, où les liposomes sont unilamellaires et d'un diamètre sensiblement prédéterminé.

9. Méthode de production de liposomes activés caractérisée en ce que les liposomes ayant des surfaces externes comprenant un certain nombre de molécules de lipide, lesdites molécules de lipide ayant des têtes polaires et des queues non polaires et au moins certaines des têtes polaires avec des groupes oxydables, sont mis en contact avec un réactif oxydant pour produire des liposomes activés par oxydation des groupes oxydables en fragments aldéhydes.

10. Méthode selon la revendication 9, oú au moins certaines desdites têtes polaires avec les groupes oxydables ont des groupes hydroxyles vicinaux et sont choisies parmi le lactosylcéramide, le galactocérébroside, le trihexosylcéramide, le phosphatidylglycérol, le phosphatidylinositol, des gangliosides et leurs mélanges.

11. Méthode selon la revendication 10, où lesdites molécules de lipide sont un mélange de lipides, le mélange contenant de la phosphatidylcholine ou de la sphingomyéline.

12. Méthode selon la revendication 10 ou la revendication 11, où lesdites molécules de lipide ayant

lesdites têtes polaires avec des groupes hydroxyles vicinaux sont en une quantité d'au moins environ 10% molaires par rapport à un total dudit mélange de lipides.

13. Méthode selon l'une quelconque des revendications 9 à 11, où lesdites molécules de lipide définissent une couche bimoléculaire et lesdits liposomes comprennent du cholestérol disposé dans ladite couche bimoléculaire.

14. Méthode selon l'une quelconque des revendications 9 à 12, où lesdites liposomes sont des vésicules unilamellaires ayant des diamètres sensiblement équivalents.

15. Méthode selon l'une quelconque des revendications 9 à 14, où ledit réactif oxydant est un périodate réactif ou est un tétraacétate de plomb.

16. Méthode selon la revendication 5, oú l'étape de mise en contact est entreprise dans un solvant polaire et ledit réactif oxydant est du périodate de sodium à un rapport par rapport aux molécules de lipide de 1,5:1 à 6:1.

17. Méthode de couplage de protéines à des liposomes en:

activant des liposomes, par une méthode selon l'une quelconque des revendications 9 à 16 et

mélangeant une protéine ayant au moins un groupe amino primaire ou secondaire auxdits liposomes activés, le mélange étant entrepris dans un solvant polaire en présence d'une quantité suffisante de cyanoborohydrure de sodium pour lier ladite protéine auxdits liposomes activés par liaison covalente.

18. Méthode selon la revendication 17, où ladite protéine a au moins environ 20 fragments de lysyle sur sa longueur.

19. Méthode selon la revendication 17, où ladite protéine est IgG ou $F[ab']_2$.

20. Méthode selon la revendication 17, la revendication 18 ou la revendication 19, où la protéine est un anticorps ayant une capacité de liaison d'un antigène déterminable d'origine avant l'étape de mélange et ladite protéine liée auxdits liposomes de l'étape de mélange a une capacité de liaison de l'antigène qui n'est pas de moins de 10% par rapport à ladite capacité de liaison de l'antigène déterminable d'origine.

21. Liposome lié par des liaisons covalentes à au moins 40µg par micromole d'une protéine biologiquement active pouvant être obtenue par une méthode selon l'une quelconque des revendications 7 à 20.